# EUROPEAN PATENT APPLICATION

(11) **EP 4 442 810 A1**
(43) Date of publication of application: **09.10.2024**
(21) Application number: 22901438.6
(22) Date of filing: 02.12.2022
(51) Int. Cl.: C12N 5/071, C12N 5/0735

(54) **CULTURE METHOD OF FISH GERM STEM CELLS**

(30) Priority: 03.12.2021 JP 2021196963
(71) Applicant: Kyushu University, National University Corporation, Nishi-ku Fukuoka-shi Fukuoka 819-0395 (JP); University of Miyazaki, Miyazaki-shi Miyazaki 889-2192 (JP)
(72) Inventor: CHAKRABORTY, Tapas, Fukuoka-shi, Fukuoka 819-0395 (JP); OHTA, Kohei, Fukuoka-shi, Fukuoka 819-0395 (JP); MATSUYAMA, Michiya, Fukuoka-shi, Fukuoka 819-0395 (JP); MOHAPATRA, Sipra, Fukuoka-shi, Fukuoka 819-0395 (JP); YAHIRO, Issei, Fukuoka-shi, Fukuoka 819-0395 (JP); MIZUMURA, Kodai, Fukuoka-shi, Fukuoka 819-0395 (JP); NAGANO, Naoki, Miyazaki-shi, Miyazaki 889-2192 (JP)
(74) Representative: J A Kemp LLP
(86) International application number: PCT/JP2022/044532
(87) International publication number: WO 2023/101008

(57) **Abstract**

The present invention provides a method for culturing a fish germ stem cell, including culturing the stem cell on a surface coated with vitronectin and in a medium containing the following 8 components:
(1) insulin,
(2) selenium,
(3) transferrin,
(4) L-ascorbic acid,
(5) FGF2,
(6) TGFβ,
(7) NaHCO₃ or KHCO₃,
(8) L-glutamine.

## Description

### [Technical Field]

The present invention relates to methods for culturing fish germ stem cells and the like. More particularly, the present invention relates to methods for culturing fish germ stem cells under serum-free and feeder-free conditions and the like.

### [Background Art]

Given the accelerated increase in the global population, serious food shortage is expected to occur in the future. As one approach to solve this problem, the construction of efficient production and management means for marine product resources has been drawing attention in recent years.

One of the means to efficiently produce marine product resources is, for example, to breed varieties that grow in a short period of time and varieties that are highly resistant to diseases.

In order to efficiently breed such preferred fish and shellfish varieties, one method is an approach using genetic modification technique such as one used in the field of animals (Non Patent Literature 1). To take this approach, a technique for stably maintaining and proliferating large amounts of fish and shellfish germ stem cells is essential.

However, compared to the field of animals, the technique for stably proliferating large amounts of germ stem cells has not been sufficiently established in the field of seafood.

### [Citation List]

### [Non Patent Literature]

### [Non Patent Literature 1]

Yoshiko Iwasaki-Takahashi et al. Commun Biol. 2020 Jun 15; 3(1):308.

### [Summary of Invention]

### [Technical Problem]

In view of the above-mentioned situation, the problem of the present invention is to provide a novel method for culturing fish germ stem cells stably in large amounts.

### [Solution to Problem]

The present inventors have conducted intensive studies of the above-mentioned problem and found that fish germ stem cells proliferate efficiently while favorably maintaining the stemness thereof, even under serum-free and feeder-free conditions, by culturing the stem cells on a surface coated with vitronectin and in a medium containing 8 specific components. Based on such finding, they conducted further studies and completed the present invention

Accordingly, the present invention provides the following.

[1] A method for culturing a fish germ stem cell, comprising culturing the fish stem cell on a surface coated with vitronectin and in a medium comprising the following 8 components:
   (1) insulin,
   (2) selenium,
   (3) transferrin,
   (4) L-ascorbic acid,
   (5) FGF2,
   (6) TGFβ,
   (7) NaHCO₃ or KHCO₃,
   (8) L-glutamine.
[2] The method of [1], wherein the medium is a serum-free medium.
[3] The method of [1] or [2], wherein the method is performed under feeder-free conditions.
[4] The method of any of [1] to [3], comprising a step of reseeding at 0.5×10⁵ to 6.5×10⁵ cells/cm² at the time of passage.
[5] A kit for culturing a fish germ stem cell, comprising the following:
   vitronectin, and
   the following 8 components:
      (1) insulin,
      (2) selenium,
      (3) transferrin,
      (4) L-ascorbic acid,
      (5) FGF2,
      (6) TGFβ,
      (7) NaHCO₃ or KHCO₃,
      (8) L-glutamine.
[6] The kit of [5], further comprising a basal medium.
[7] The kit of [5] or [6], further comprising sodium chloride when (7) is KHCO₃.

### [Advantageous Effects of Invention]

According to the present invention, fish germ stem cells can be proliferated extremely efficiently under serum-free and feeder-free conditions while favorably maintaining the stemness thereof.

### [Brief Description of Drawings]

[Fig. 1]

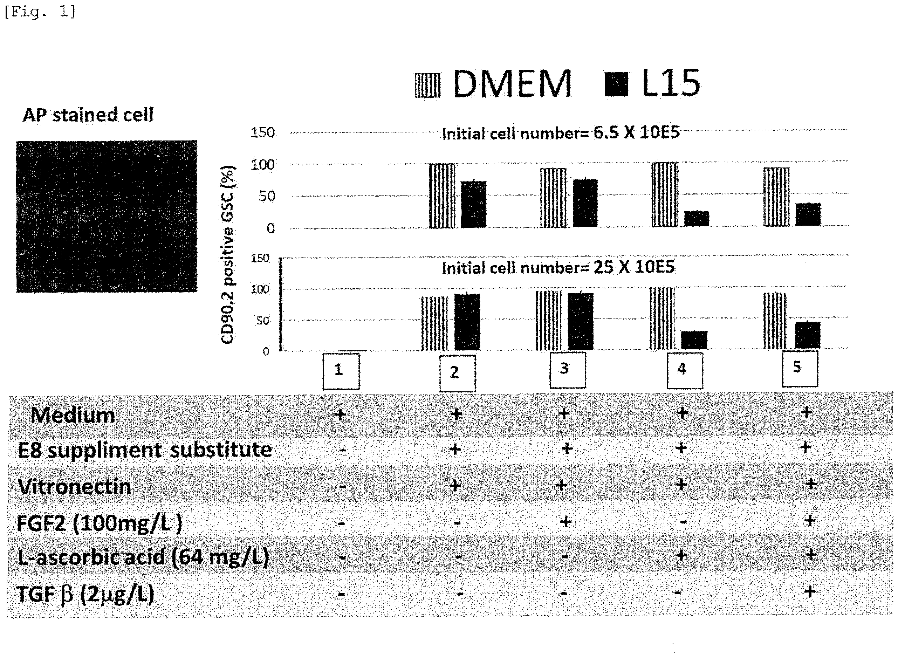

   Fig. 1 shows a photograph (upper left) of germ stem cells when alkaline phosphatase staining (AP stain) was performed, and diagrams presenting the percentage of CD90.2-positive germ stem cells when germ stem cells of Chub mackerel were cultured in DMEM or L15 medium supplemented with various components in the presence or absence of vitronectin (medium 1 to 5 (DMEM or L15), bar graph in the upper panel: cell number on seeding 6.5×10⁵ cells, bar graph in the lower panel: cell number on seeding 25×10⁵ cells) .
[Fig. 2]

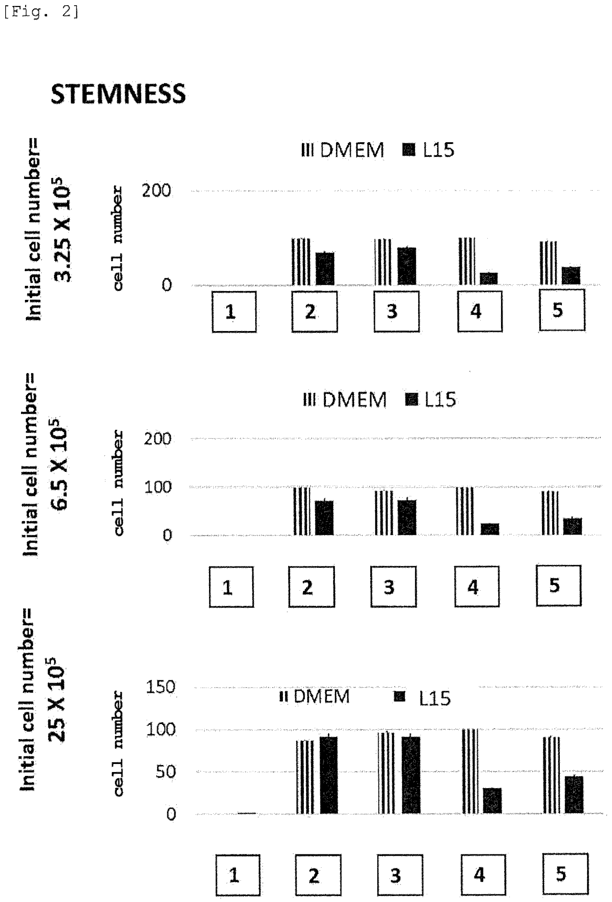

   Fig. 2 shows diagrams presenting the ratio of stemness of germ stem cells of Chub mackerel after culturing the germ stem cells in medium 1 to 5 (DMEM or L15) by varying the number of cells on seeding.
[Fig. 3]

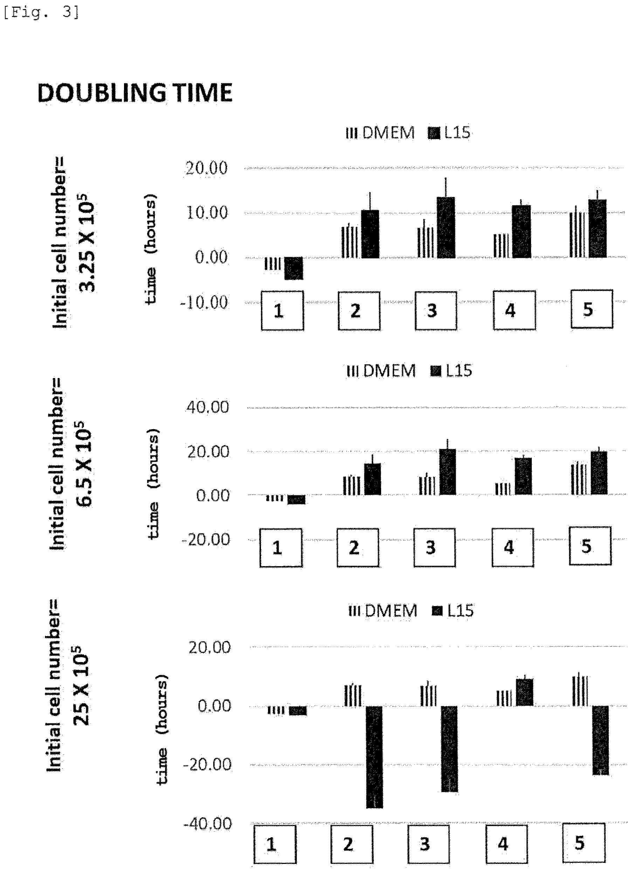

   Fig. 3 shows diagrams presenting the doubling time of germ stem cells of Chub mackerel when the germ stem cells were cultured in medium 1 to 5 (DMEM or L15) by varying the number of cells on seeding.
[Fig. 4]

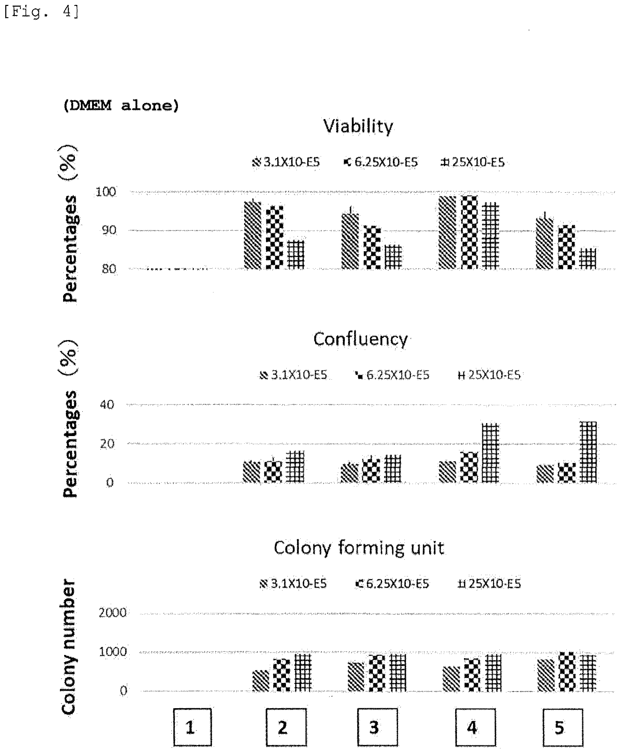

   Fig. 4 shows diagrams presenting the viability, confluency, and CFU of germ stem cells of Chub mackerel when the germ stem cells were cultured in medium 1 to 5 (DMEM alone) by varying the number of cells on seeding.
[Fig. 5]

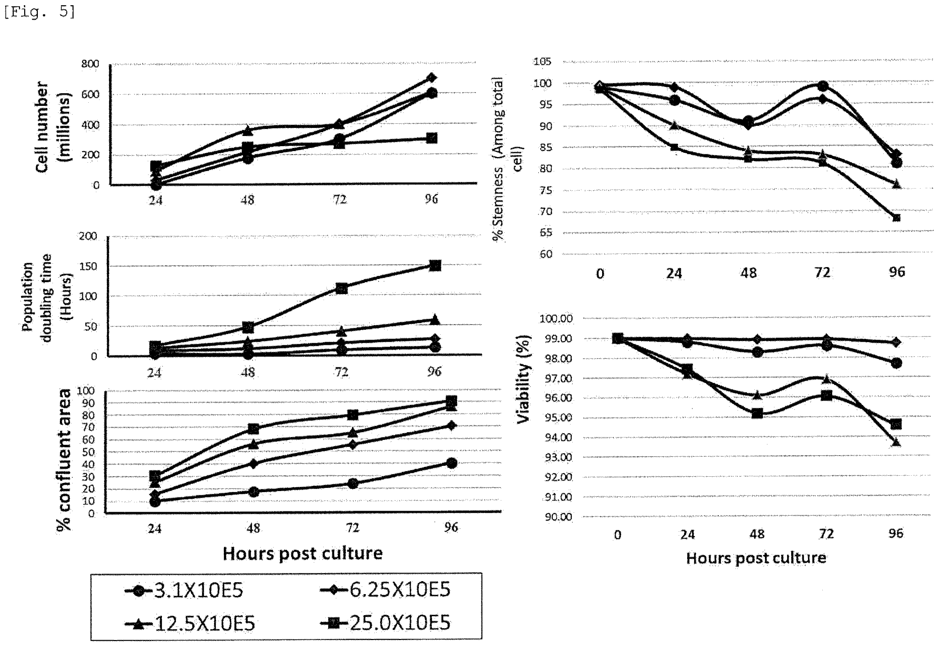

   Fig. 5 shows diagrams presenting the confirmation results of the cell number, doubling time, confluent area, stemness, and viability over time for 4 days, when the cell density was changed on passage during culture of germ stem cells of Chub mackerel by using medium 4 (DMEM/vitronectin coating).
[Fig. 6]

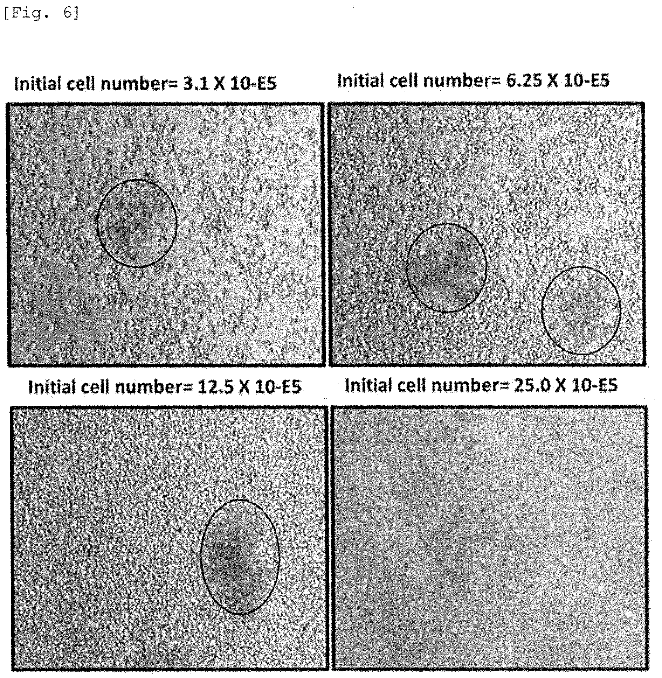

   Fig. 6 shows photographs of cells 4 days after passage, when the cell density was changed on passage during culture of germ stem cells of Chub mackerel by using medium 4 (DMEM/vitronectin coating).
[Fig. 7]

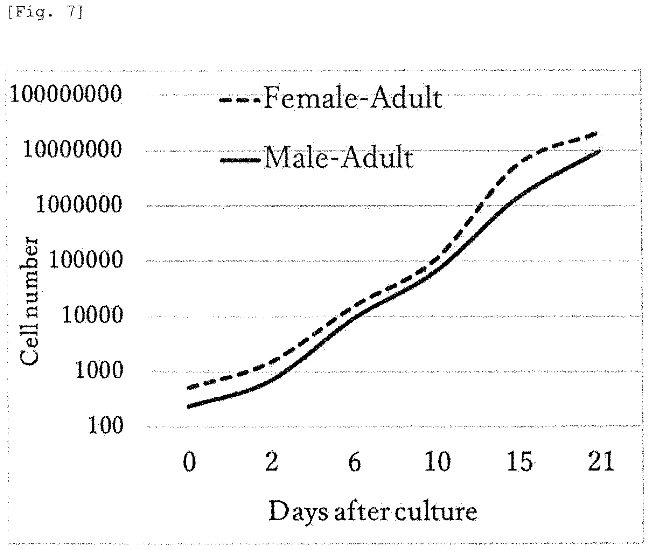

   Fig. 7 is a graph showing the proliferation efficiency when germ stem cells taken from female and male Chub mackerel were cultured under the conditions of a medium containing 8 specific components and coating with vitronectin.
[Fig. 8]

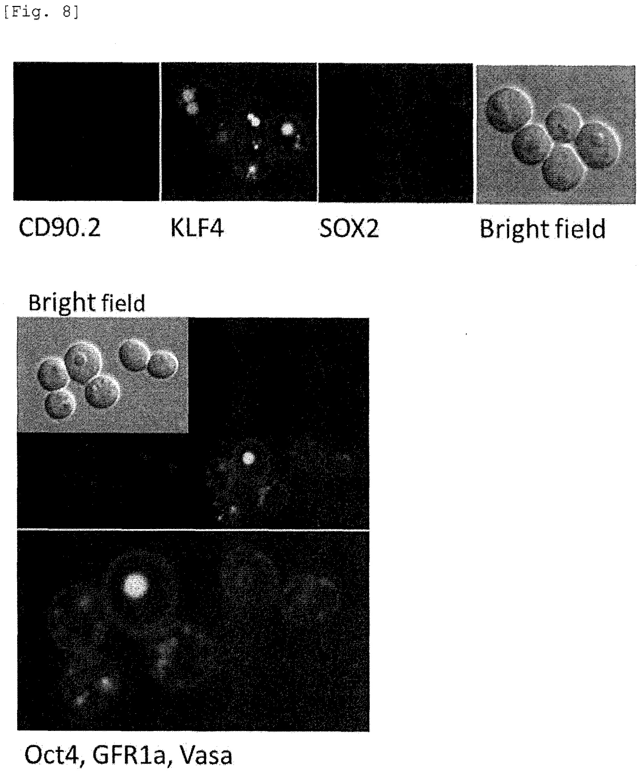

   Fig. 8 shows photographs of the expression of various cell markers (CD90.2, KLF4, SOX2, Oct4, GFR1a, and Vasa) when germ stem cells taken from female and male Chub mackerel were cultured under the conditions of a medium containing 8 specific components and coating with vitronectin.
[Fig. 9]

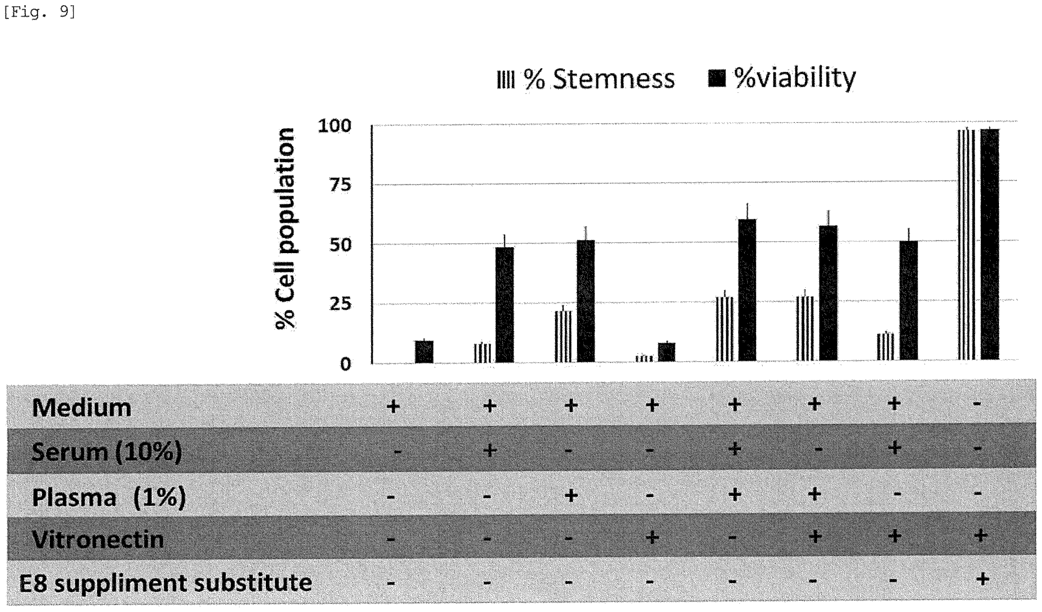

   Fig. 9 shows diagrams presenting the stemness and viability of germ stem cells of killifish cultured under the various conditions.
[Fig. 10]

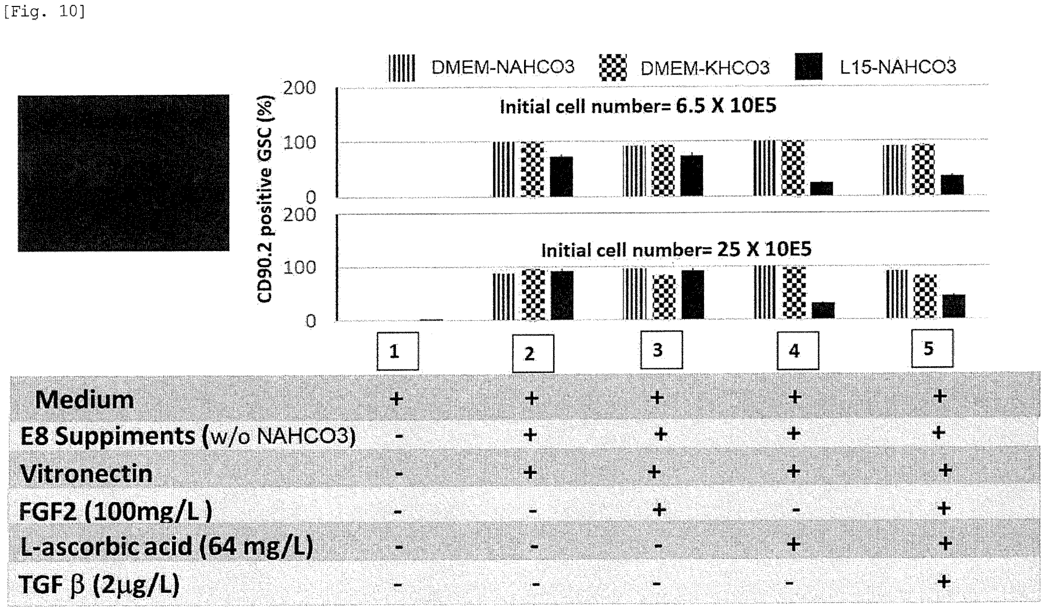

   Fig. 10 shows diagrams indicating that NaHCO₃ to be added to DMEM medium can be replaced with KHCO₃, in the culture of fish germ stem cells.
[Fig. 11]

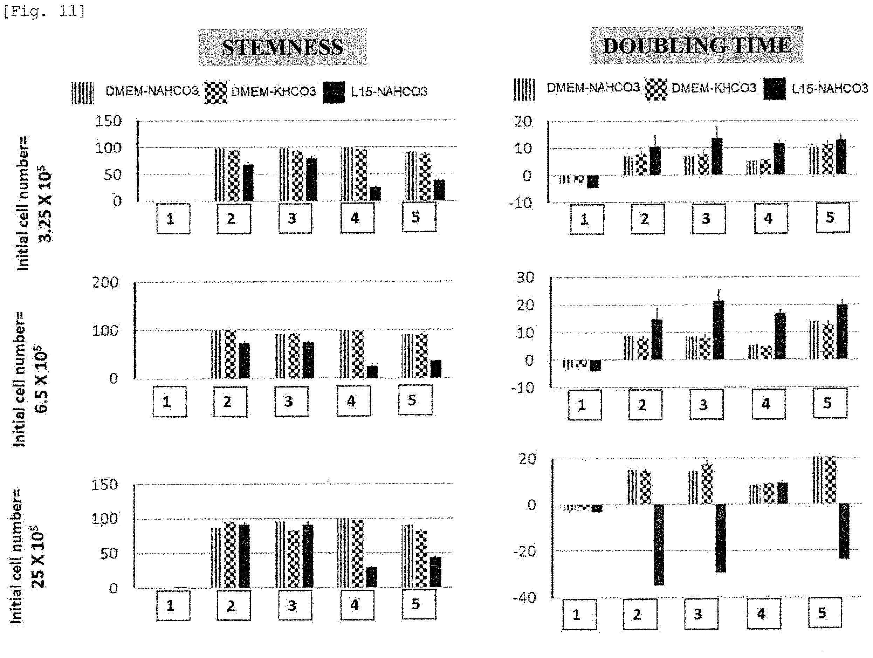

   Fig. 11 shows graphs presenting the stemnness and doubling time, when the cell density was changed at the time of passage under each condition of Example 7.
[Fig. 12]

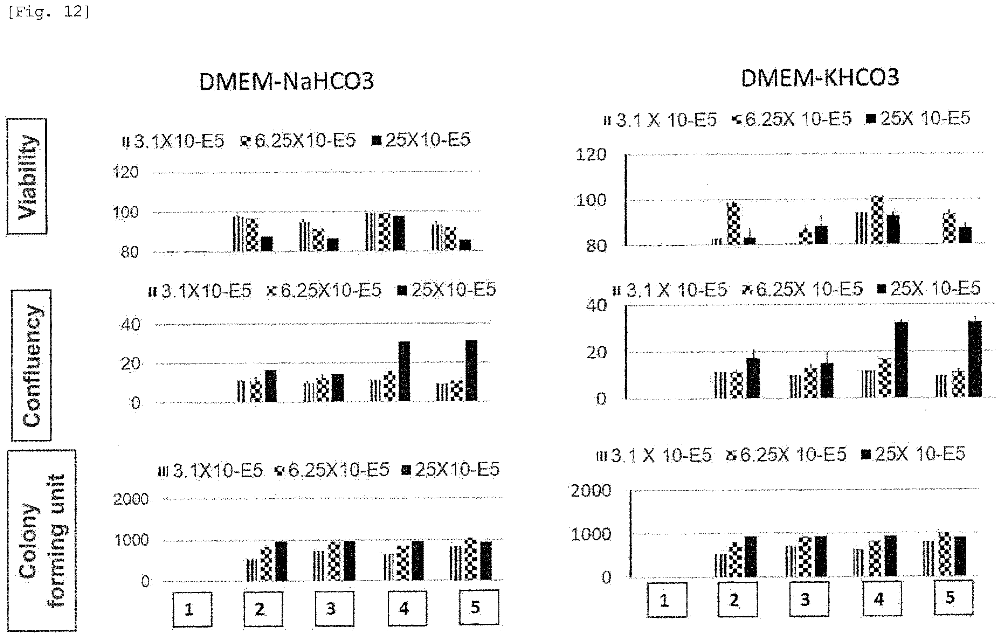

   Fig. 12 shows graphs presenting the viability, confluency, and CFU of fish germ stem cells under each condition of Example 9.
[Fig. 13]

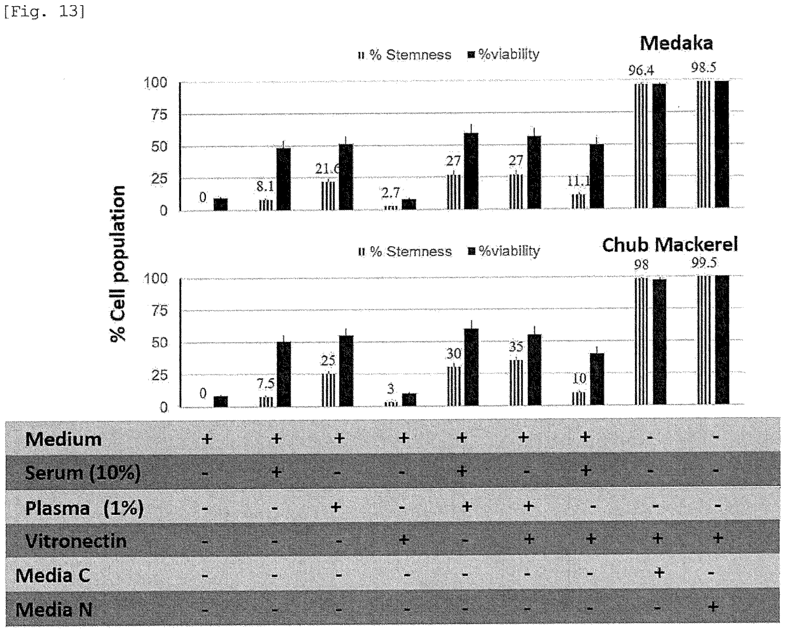

   Fig. 13 shows graphs presenting the stemness and viability of germ stem cells of killifish and Chub mackerel when they were cultured under various conditions.
[Fig. 14]

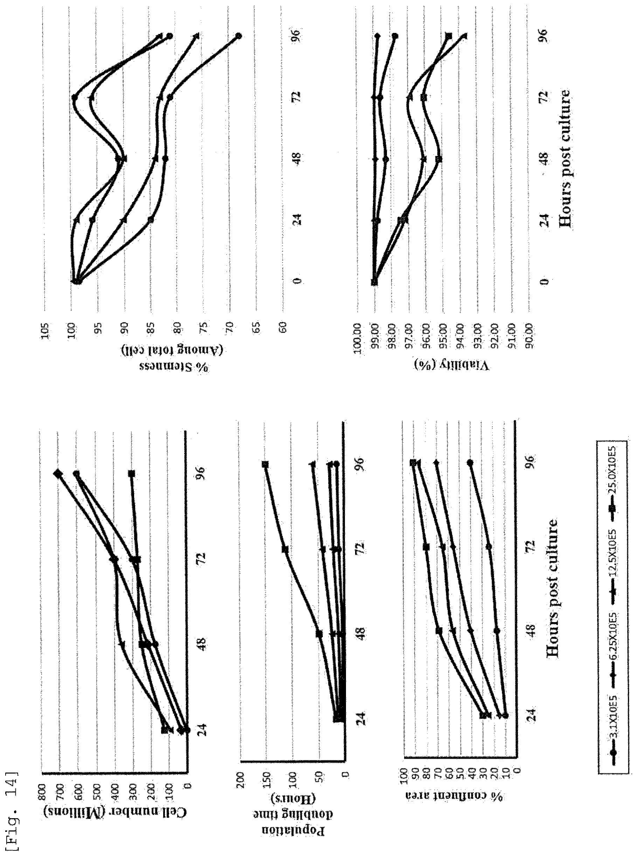

   Fig. 14 shows graphs presenting the cell number, population doubling time, confluency (confluent area), stemness, and viability when germ stem cells of Chub mackerel were cultured at four cell densities using Media C and vitronectin.
[Fig. 15]

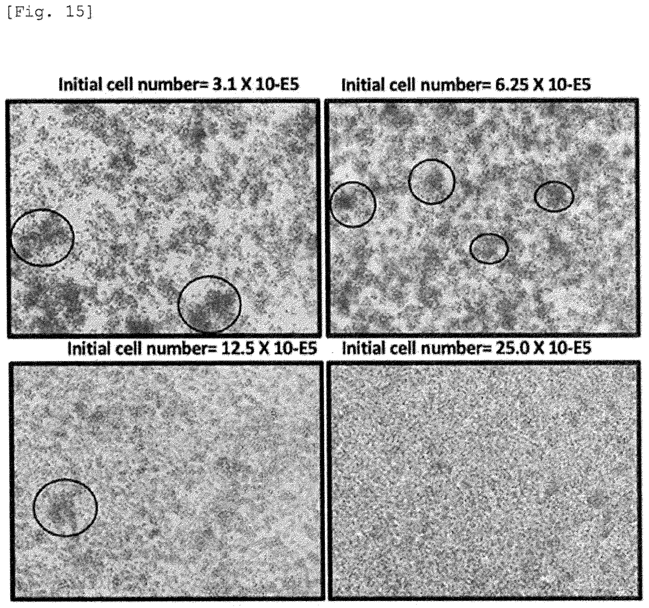

   Fig. 15 shows photographs of cell populations when germ stem cells of Chub mackerel were cultured at four cell densities using Media C and vitronectin.
[Fig. 16]

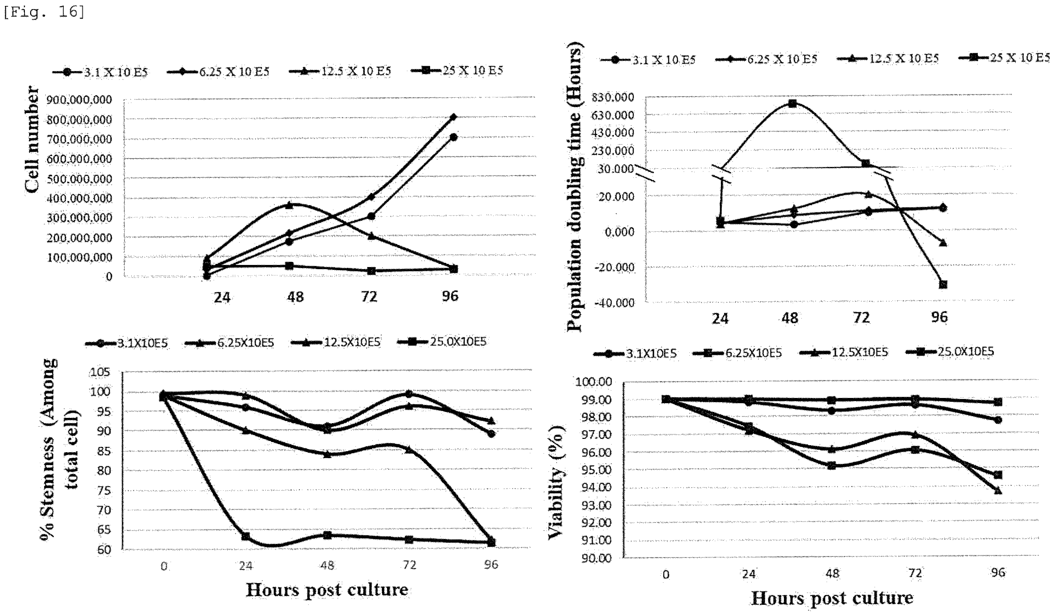

   Fig. 16 shows graphs presenting the cell number, population doubling time, stemness, and viability when germ stem cells of Chub mackerel were cultured at four cell densities using Media N and vitronectin.
[Fig. 17]

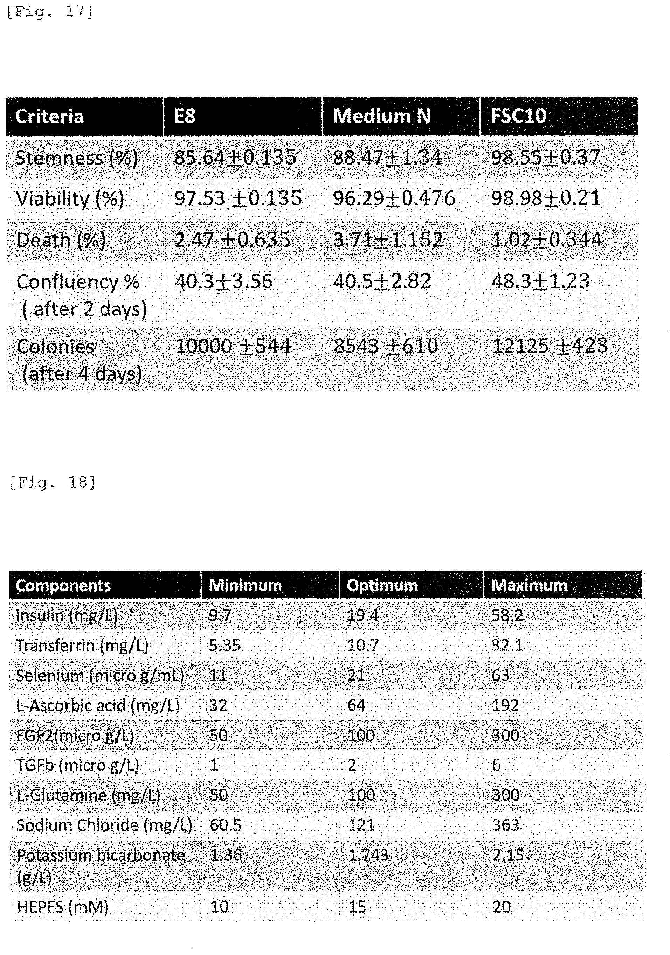

   Fig. 17 shows that a medium (FSC10) obtained by additionally adding selenium and adding sodium chloride (NaCl) to Media N is superior to Media N in terms of stemness, viability, mortality, confluency, and colony number.
[Fig. 18]
   Fig. 18 shows preferred concentration ranges of the active ingredients of FSC10.

### [Description of Embodiments]

The present invention is described in detail below.

### 1. Culture method of fish germ stem cells

The present invention provides a method for culturing a fish germ stem cell, including culturing the fish stem cell on a surface coated with vitronectin and in a medium containing 8 specific components ((1) insulin, (2) selenium, (3) transferrin, (4) L-ascorbic acid, (5) FGF2, (6) TGFβ, (7) NaHCO₃ or KHCO₃, and (8) L-glutamine) (hereinafter sometimes referred to as "the method of the present invention").

In the method of the present invention, all 8 components contained in the medium can be produced by methods known per se, and commercially available products may also be used. The species from which insulin, transferrin, FGF2, and TGFβ are derived is not particularly limited as long as the desired effect of the present invention is obtained, and it is preferably human. These components may be of biological origin, or may be recombinant proteins. When (7) is NaHCO₃, commercially available Essential 8^{™} supplement (Thermo scientific, Catalogue no A1517001), which is a mixture of components (1) to (8), is also preferably used.

The amounts of these 8 components to be added to the medium are not particularly limited as long as the desired effect of the present invention is obtained. In one embodiment, the following amounts can be adopted.
(1) insulin: 0.1 - 200 mg/L (preferably, 1 - 150 mg/L, 5 - 100 mg/L, 5 - 90 mg/L, 10 - 80 mg/L, or 10 - 70 mg/L)
(2) selenium: 0.1 - 100 µg/L (preferably, 1 - 90 µg/L, 5 - 80 µg/L, 5 - 70 µg/L, 10 - 60 µg/L, or 10 - 50 µg/L)
(3) transferrin: 0.1 - 200 mg/L (preferably, 1 - 150 mg/L, 1 - 100 mg/L, 1 - 90 mg/L, 5 - 80 mg/L, or 5 - 70 mg/L)
(4) L-ascorbic acid: 1 - 500 mg/L (preferably, 1 - 400 mg/L, 5 - 300 mg/L, 10 - 250 mg/L, 10 - 200 mg/L, or 10 - 150 mg/L)
(5) FGF2: 1 - 500 µg/L (preferably, 1 - 400 µg/L, 5 - 350 µg/L, 10 - 300 µg/L, 20 - 250 µg/L, or 50 - 250 µg/L)
(6) TGFβ: 0.01 - 20 µg/L (preferably, 0.1 - 10 µg/L, 0.5 - 10 µg/L, 0.5 - 8 µg/L, 0.7 - 8 µg/L, or 0.8 - 6 µg/L)
(7) NaHCO₃: 0.01 - 20 g/L (preferably, 0.1 - 10 g/L, 0.5 - 10 g/L, 0.5 - 8 g/L, 0.7 - 8 g/L, or 0.8 - 6 g/L)
(8) L-glutamine: 1 - 500 mg/L (preferably, 1 - 400 mg/L, 5 - 350 mg/L, 10 - 300 mg/L, 20 - 250 mg/L, or 50 - 250 mg/L)

In one embodiment, NaHCO₃ may be replaced by the same amount of KHCO₃.

In another embodiment, when KHCO₃ is used in the method of the present invention, it is preferable to add sodium chloride (NaCl). In one embodiment with the addition of NaCl, the following concentrations can be preferably used:
(1) insulin: 10 - 60 mg/L (preferably, 10 - 58 mg/L, 10 - 50 mg/L, 11 - 45 mg/L, 15 - 30 mg/L, or 15 - 25 mg/L)
(2) selenium: 11 - 63 µg/L (preferably, 13 - 60 µg/L, 15 - 50 µg/L, 17 - 40 µg/L, 18 - 30 µg/L, or 19 - 25 µg/L)
(3) transferrin: 5 - 32 mg/L (preferably, 5.5 - 30 mg/L, 5.5 - 28 mg/L, 6 - 26 mg/L, 8 - 25 mg/L, or 9 - 15 mg/L)
(4) L-ascorbic acid: 32 - 192 mg/L (preferably, 40 - 150 mg/L, 45 - 100 mg/L, 50 - 90 mg/L, 55 - 80 mg/L, or 57 - 75 mg/L)
(5) FGF2: 50 - 300 µg/L (preferably, 60 - 200 µg/L, 70 - 180 µg/L, 75 - 150 µg/L, 80 - 120 µg/L, or 90 - 110 µg/L)
(6) TGFβ: 1 - 6 µg/L (preferably, 1.1 - 5 µg/L, 1.5 - 4 µg/L, 1.6 - 3.5 µg/L, 1.7 - 2.8 µg/L, or 1.8 - 2.6 µg/L)
(7) KHCO₃: 1.36 - 2.15 g/L (preferably, 1.4 - 2.00 g/L, 1.5 - 1.90 g/L, 1.5 - 1.85 g/L, 1.6 - 1.83 g/L, or 1.65 - 1.80 g/L)
(8) L-glutamine: 50 - 300 mg/L (preferably, 60 - 200 mg/L, 80 - 180 mg/L, 70 - 150 mg/L, 80 - 120 mg/L, or 90 - 110 mg/L)
(9) NaCl: 60.5 - 363 mg/L (preferably, 65 - 350 mg/L, 80 - 300 mg/L, 90 - 200 mg/L, 100 - 150 mg/L, or 110 - 130 mg/L)

Vitronectin (or may be vitronectin fragment as long as the functions thereof are maintained) to be used in the present invention may be produced by a method known per se, or a commercially available product may also be used. The species from which vitronectin or a fragment thereof is derived is not particularly limited as long as the desired effect of the present invention is obtained, and it is preferably human.

Coating of the surface of culture containers such as well, dish, and the like with vitronectin can also be performed by a method known per se. As one embodiment, vitronectin is dispersed in PBS, and the PBS is dispensed to a culture container and incubated at 37°C for 1 hr, whereby the inner surface of the culture container can be coated with vitronectin.

The medium that can be used in the method of the present invention is not particularly limited as long as the desired effect of the present invention is obtained, and any basal medium can be used. In one embodiment, the following media can be used preferably. Dulbecco's Modified Eagle's medium (DMEM), Ham's Nutrient Mixture F12 medium, DMEM/F12 medium, McCoy's 5A medium, Eagle's Minimum Essential medium (EMEM), alpha Modified Eagle's Minimum Essential medium (αMEM), MEM medium (Minimum Essential medium), RPMI1640 medium, Iscove's Modified Dulbecco's medium (IMDM), MCDB131 medium, Williams' medium E, IPL41 medium, Fischer's medium, StemPro34 (manufactured by Invitrogen Corp.), X-VIVO 10 (manufactured by Cambrex), X-VIVO 15 (manufactured by Cambrex), HPGM (manufactured by Cambrex), StemSpan H3000 (manufactured by STEMCELL Technologies), StemSpan SFEM (manufactured by STEMCELL Technologies), StemlineII (manufactured by Sigma Ltd. Aldrich), QBSF-60 (manufactured by Quality Biological), StemProhESCSFM (manufactured by Invitrogen Corp.), Essentials (registered trade mark) medium (manufactured by Gibco), Essentials (registered trade mark) medium (manufactured by Gibco), Essentials (registered trade mark) Flex medium (manufactured by Thermo Fisher Scientific), StemFlex medium (manufactured by Thermo Fisher Scientific), StemScale (registered trade mark) PSC Suspension medium (manufactured by Thermo Fisher Scientific), mTeSR1 or 2 or Plus medium (manufactured by STEMCELL Technologies), ReproFF or ReproFF2 (manufactured by REPROCELL), PSGro hESC/iPSC medium (manufactured by System Biosciences), NutriStem (registered trade mark) medium (manufactured by Biological Industries), MSC NutriStem (registered trade mark) XF medium (manufactured by Biological Industries), CSTI-7 medium (manufactured by Cell Science & Technology Institute, Inc.), MesenPRO RS medium (manufactured by Gibco), MF-medium (registered trade mark) mesenchymal stem cellular proliferation medium (manufactured by TOYOBO), mesenchymal stem cell serum-free medium (manufactured by Fukoku), Mesenchymal Stem Cell Growth medium 2 (manufactured by PromoCell), Sf-900II (manufactured by Invitrogen Corp.), Opti-Pro (manufactured by Invitrogen Corp.), StemFit (registered trade mark) AK02N or Basic02 or AK03N or Basic03 or Basic04 medium (manufactured by Ajinomoto Health & Nutrition Supply), STEMUP medium (manufactured by Nissan chemical Corporation), L15 medium, and the like. Preferred may be DMEM/F12 (Ham) 1:1 medium.

It is sometimes preferred to stabilize the pH of the medium used for culturing fish germ stem cells at around 7 to 7.5. In such cases, the pH of the medium can be adjusted using methods known per se. In one embodiment, the pH can be adjusted by adding an appropriate amount of a buffer such as HEPES to the medium, but the method is not limited thereto.

Also, in one embodiment, known medium additives such as amino acids (essential amino acid, non-essential amino acid, etc.), antibiotics (gentamicin, etc.), minerals (calcium, magnesium, etc.), and buffers (HEPES, etc.) may also be added as appropriate to the medium, in addition to the above-mentioned components.

Various culture conditions in the method of the present invention are not particularly limited as long as the desired effects of the present invention are obtained. As one embodiment, the culture temperature is generally 25 - 39°C (preferably 30 - 37°C). The CO₂ concentration in the culture atmosphere is generally 1 to 10% by volume, preferably 2 to 5% by volume. In addition, the culture period may be appropriately set depending on the purpose of culture. Using the present invention, culture may be possible for 1 to 100 days or more than 100 days. The frequency of medium replacement may be, but is not limited to, every day, once every 2 days, once every 3 days, once every 4 days, once every 5 days, once every 6 days, or once every 7 days.

The species of fish to which the method of the present invention can be applied is not particularly limited. Examples include Beloniformes (e.g., killifish, needlefish, mackerel pike, etc.), Clupeiformes (e.g., sardine, Pacific herring, silver-stripe round herring, etc.), Perciformes (e.g., wrasse, mackerel, scorpion fish, etc.), and the like. Killifish, anchovy, Siebold's wrasse, and Chub mackerel are preferred, and Chub mackerel is particularly preferred.

Serum may be included in the method of the present invention. In a preferred embodiment, the medium used in the present invention may be a serum-free medium. By not using serum, various disadvantages of using serum (e.g., high cost, problems relating to standardization, contamination, etc.) can be avoided, and germ stem cells with high quality can be stably produced at a comparatively low cost.

In one embodiment, the method of the present invention can also be performed under feeder-free conditions. By culturing germ stem cells under feeder-free conditions, contamination with feeder cells can be preferably avoided when collecting the germ stem cells after culture.

In one embodiment, the method of the present invention may proliferate germ stem cells in large quantities while maintaining them for a long period of time by passaging the germ stem cells. For passaging, a method known per se may be used. In one embodiment, it may be preferable to optimize cell density at the time of passage. The preferred cell density is generally 0.5×10⁵ - 6.5×10⁵ cells/cm², more preferably 1×10⁵ - 5.0×10⁵ cells/cm², further preferably 2×10⁵ - 4.5×10⁵ cells/cm², but not limited to these.

### 2. Kit for culturing fish germ stem cell

The present invention also provides a kit for culturing a fish germ stem cell, comprising the following (hereinafter sometimes referred to as "the kit of the present invention"):
vitronectin, and the following 8 components: (1) insulin, (2) selenium, (3) transferrin, (4) L-ascorbic acid, (5) FGF2, (6) TGFβ, (7) NaHCO₃ or KHCO₃, (8) L-glutamine.

In one embodiment, the kit of the present invention may further contain a basal medium.

The basal medium, vitronectin, 8 specific components, species of fish, and the like to be contained in the kit of the present invention are the same as those explained for the method of the present invention.

Vitronectin, 8 specific components, and the basal medium to be contained in the kit of the present invention may be included in the kit each in the form of being enclosed in a separate container. The 8 specific components may be respectively enclosed in separate containers, or two or more components may be enclosed in one container. All 8 components may be mixed and enclosed in one container.

In one preferred embodiment, vitronectin and the 8 specific components included in the kit of the present invention can be provided in the form of being dissolved or dispersed in an appropriate solution. In addition, the basal medium can be provided in the form of a liquid medium.

As explained in the method of the present invention, NaHCO₃ can be replaced with the same molar amount of KHCO₃. Therefore, in one embodiment, NaHCO₃ may be replaced with KHCO₃.

In another embodiment, when the kit of the present invention contains KHCO₃, it is preferable to include sodium chloride (NaCl) as an additional component. According to the kit of the present invention, the method of the present invention can be performed conveniently. Therefore, the kit of the present invention can also be referred to as a kit for practicing the method of the present invention.

The present invention is explained more specifically in the following by referring to Examples. However, the present invention is not limited at all by these Examples.

### [Example]

### [Experimental Example 1] Isolation of germ stem cell

The germ stem cells of Chub mackerel and killifish used in the following Examples were isolated by a method known per se. Briefly, they were isolated by the following procedure.

Gonads were collected under sterile conditions and incubated in DPBS containing 0.001% tetracycline hydrochloride (DPBS-AB) for at least 2 hr. The gonads were then washed at least three times with calcium-free DPBS-AB, and then the gonads were minced into 2-5 mm³ pieces. They were washed with 3 mL of Accumax per 1 g of tissue and the remaining DPBS was removed. 12 mL of Accumax per 1 g of tissue was added and all contents were transferred to a disposable 10 cm dish. They were incubated at room temperature with occasional stirring with a glass Pasteur pipette until a homogeneous mixture was obtained (at least 2 hr for ovary and at least 3 hr for testis). The obtained mixture was filtered through a mesh with a pore size of 100 µm, and then through meshes with a pore size of 40 µm and 20 µm. The filtrate was centrifuged at 1400 g for 30 min at room temperature. Cell pellets were washed at least three times with DPBS-BSA and then one more time with the medium to be used next. Finally, the washed cell pellets were dispersed in the medium to be used next in an amount of 1 mL per 0.1 g of pellet. Then, germ stem cells were isolated from the dispersed cell population by performing magnetic separation of germ stem cells using a rat anti-CD90.2 antibody conjugated with magnetic beads.

### [Experimental Example 2] Preparation of medium

Using DMEM/F12 (Ham) 1:1 medium (hereinafter sometimes to be simply referred to as DMEM medium) or L15 medium as a base, 8 types of media having the components of the following Table 1 and Table 2 were prepared.

**[Table 1]**

| medium based on DMEM/F12 (ham) 1:1 medium | | | | |
|---|---|---|---|---|
| medium name (basal medium to be the base) | Medium 2 (DMEM) | Medium 3 (DMEM) | Medium 4 (DMEM) | Medium 5 (DMEM) |
| DMEM/F12 (Ham) 1:1 | Make up to 1 L | Make up to 1 L | Make up to 1 L | Make up to 1 L |
| insulin | 19.4 mg | 19.4 mg | 19.4 mg | 19.4 mg |
| selenium | 0.014 mg | 0.014 mg | 0.014 mg | 0.014 mg |
| transferrin | 10.7 mg | 10.7 mg | 10.7 mg | 10.7 mg |
| L-ascorbic acid | 64 mg | 64 mg | 128 mg | 128 mg |
| FGF2 | 0.1 mg | 0.2 mg | 0.1 mg | 0.2 mg |
| TGFb | 0.002 mg | 0.002 mg | 0.002 mg | 0.004 mg |
| L-glutamine | 100 mg | 100 mg | 100 mg | 100 mg |
| NaHCO₃ | 1743 mg | 1743 mg | 1743 mg | 1743 mg |
| HEPES | 15 mM | 15 mM | 15 mM | 15 mM |
| gentamicin | 100 mg | 100 mg | 100 mg | 100 mg |

**[Table 2]**

| medium based on L15 medium | | | | |
|---|---|---|---|---|
| medium name (basal medium to be the base) | Medium 2 (L15) | Medium 3 (L15) | Medium 4 (L15) | Medium 5 (L15) |
| L15 | Make up to 1 L | Make up to 1 L | Make up to 1 L | Make up to 1 L |
| insulin | 19.4 mg | 19.4 mg | 19.4 mg | 19.4 mg |
| selenium | 0.014 mg | 0.014 mg | 0.014 mg | 0.014 mg |
| transferrin | 10.7 mg | 10.7 mg | 10.7 mg | 10.7 mg |
| L-ascorbic acid | 64 mg | 64 mg | 128 mg | 128 mg |
| FGF2 | 0.1 mg | 0.2 mg | 0.1 mg | 0.2 mg |
| TGFb | 0.002 mg | 0.002 mg | 0.002 mg | 0.004 mg |
| L-glutamine | 100 mg | 100 mg | 100 mg | 100 mg |
| NaHCO₃ | 1743 mg | 1743 mg | 1743 mg | 1743 mg |
| HEPES | 15 mM | 15 mM | 15 mM | 15 mM |
| gentamicin | 100 mg | 100 mg | 100 mg | 100 mg |

### [Example 1] Examination of medium components suitable for culturing fish germ stem cells - 1

The germ stem cells of Chub mackerel prepared in Experimental Example 1 were cultured in a vitronectin-coated container by using the 8 types of media prepared in Experimental Example 2, and the percentage of CD90.2-positive cells after culture was confirmed. CD90.2 positivity indicates the presence of stemness. The number of cells at the start of culture was 6.5×10⁵ cells or 25×10⁵ cells (the area of the culture dish used was 1.9 cm²). As a negative control, germ stem cells were cultured using DMEM medium or L15 medium in a container not coated with vitronectin. The cells were cultured in a container not coated with vitronectin and in DMEM medium or L15 medium. The cells were cultured in a CO₂ incubator (Panasonic) under the conditions of culture temperature 27 - 33°C, CO₂ concentration 2 - 5%. The incubator was humidified with sterile distilled water supplemented with 0.0001% sodium lauryl sulfate (Nacalai). The incubator was UV sterilized for 15 min every 4 days and washed with H₂O₂ monthly. CD90.2-positive cells were subjected to immune cell staining using a fluorescently labeled antibody against CD90.2 and analyzed using a cell analyzer (Sony EC800).

As shown in Fig. 1, the CD90.2-positive cell was scarcely or not confirmed under the conditions of not containing E8 supplement and the like and not coating with vitronectin (Fig. 1 "1"). On the other hand, many CD90.2-positive cells were confirmed under the conditions of containing E8 and culturing the germ stem cells in a vitronectin-coated container. Also, a tendency to obtain a larger number of CD90.2-positive cells was confirmed when DMEM was used as the basal medium.

### [Example 2] Examination of medium components suitable for culturing fish germ stem cells - 2

The germ stem cells of Chub mackerel prepared in Experimental Example 1 were cultured in a vitronectin-coated container by using the 8 types of media (and 2 types of media as negative targets) prepared in Experimental Example 2, and the stemness and doubling time after culture were confirmed. The number of cells at the start of culture was 3.25×10⁵ cells, 6.5×10⁵ cells, or 25×10⁵ cells (the area of the culture dish used was 1.9 cm²). As a negative control, germ stem cells were cultured using DMEM medium or L15 medium in containers not coated with vitronectin. The undifferentiated state and doubling time by culture for 24 hr were confirmed. In the experiment, n=6. The stemness was calculated using CD90.2 staining and the cell analysis was performed using Sony EC800. Other respective experiment conditions were the same as in Example 1. For the doubling time, initial cell number (N0) at the time point of initial time (t0) and cell number (N) after t hours were determined, and the doubling time was calculated using the following formula: cell population doubling time (CPDT) = (t- t0) / (3.32* (LOG10(N)-LOG10(N0) )

The results are shown in Fig. 2 (stemness) and Fig. 3 (doubling time). The stemness could be maintained, as shown in Fig. 2, even when any of DMEM medium and L15 medium was used as the basal medium. Similar to Example 1, the stemness could be maintained more efficiently when DMEM medium was used as the basal medium.

The doubling time, as shown in Fig. 3, tended to become shorter than L15 when DMEM was used as the basal medium. The doubling time tended to vary depending on the number of cells initially seeded. (Negative values indicate decreased cell number).

From the above results, the stemness could be maintained under specific conditions, and efficient proliferation of germ stem cells could be achieved under specific conditions, even when either DMEM medium or L15 medium was used as the basal medium.

### [Example 3] Examination of medium components suitable for culturing fish germ stem cells - 3

Under the same conditions as in Example 1 except that the basal medium was DMEM alone, the germ stem cells of Chub mackerel prepared in Experimental Example 1 were cultured, and the cell survival, confluency, and CFU (Colony Forming Unit) were confirmed 24 hr after the start of culture. The number of cells at the start of culture was 3.1×10⁵ cells, 6.25×10⁵ cells, or 25×10⁵ cells (the area of the culture dish used was 1.9 cm²), n=6. The viability was calculated using propidium iodide and Hoechst staining and the cell analysis was performed using Sony EC800. Confluency was calculated by the ratio of the area covered by the cells to the total available area of the medium. Colonies (>50 µ) were counted individually. The results are shown in Fig. 4.

As shown in Fig. 4, it was shown that germ stem cells can be cultured with any medium composition of media 2 to 4 (DMEM) when a vitronectin-coated container is used. In addition, it was shown that the conditions of medium 4 (DMEM) are particularly preferred.

### [Example 4] Examination of optimal cell density at the time of passage - 1

Using medium 4 (DMEM) of Example 1, the optimal cell density at the time of passage was examined. The number of cells at the time of passage was 3.1×10⁵ cells, 6.25×10⁵ cells, 12.5×10⁵ cell, or 25×10⁵ cells (the area of the culture dish used was 1.9 cm²) and passage was performed by the following procedure: passage was performed at 4-day intervals. Between each passage, the upper layer of the spent medium (70-80% of the total medium) that does not contain cell was discarded. The cells were then gently suspended using a pipette. The cell suspension was collected in a 50 ml conical tube and centrifuged at 1400×g for 10 min. The cell pellet was carefully suspended in a fresh final medium. The cell number was counted and seeded in a container coated with vitronectin. After 46 to 52 hr of passage, the cell number, doubling time, stemness, and viability were calculated. The cell number was calculated using a cell analyzer. The doubling time, stemness, and viability were calculated by the same methods as above. The results are shown in Fig. 5 (cell number, doubling time, stemness, viability) and Fig. 6 (photograph of cell populations after culture under each condition after 96 hr of passage).

As shown in Fig. 5 and Fig. 6, it was shown that efficient passage is possible by setting a comparatively small cell density at the time of passage.

### [Example 5] Cell proliferation rate and detection of cell marker after proliferation

The germ stem cells of Chub mackerel prepared in Experimental Example 1 were cultured in a vitronectin-coated container and in a DMEM/F12 (Ham) 1:1 medium containing insulin (19.4 mg/L), selenium (14 µg/L), transferrin (10.7 mg/L), L-ascorbic acid (64 mg/L), FGF2 (200 µg/L), TGFβ (2 µg/L), NaHCO3 (1.743 g/L), L-glutamine (100 mg/L), and HEPES (15 mM), and the cell proliferation rate and the expression state of cell markers (CD90.2, KLF, OCT4, GFR1a and VASA) were confirmed. The cell markers were confirmed using Live cell immunohistochemistry. The cells were passaged every 4 days. The results are shown in Fig. 7 (cell number), Fig. 8 (cell marker expression), and Table 3 (cell marker expression level).

**[Table 3]**

| | AP live stain | OD90.2 | Klf4 | Oct4 | Sox2 | Vasa | GFR1a | SOX9 |
|---|---|---|---|---|---|---|---|---|
| Passage 10 | 99.5 ±0.75 | 98,7± 1.1 | 96.4± 1.7 | 99.5± 0.5 | 0 | 0 | 54.2± 3.1 | 0 |
| Passage 15 | 98.5±0.75 | 98.8±0.97 | 97.5± 1.6 | 98.7± 0.8 | 0 | 0 | 53.4± 2:3 | 0 |
| Passage 20 | 98.4±1.23 | 99.1±0.92 | 98.4± 1.4 | 99.3± 0.6 | 0 | 0 | 45.4± 2.7 | 0 |
| Passage 25 | 98.5±1.35 | 96.4±1.75 | 95.7± 1.1 | 97.2± 1.4 | 0 | 2.1±1.4 | 50.2± 2.9 | 0 |

As shown in Fig. 7, the cell numbers of both the female-derived germ stem cells and the male-derived germ stem cells increased over time. As shown in Fig. 8, the cultured germ stem cells expressed cell markers such as CD90.2, KLF4, OCT4, GFR1a, VASA, and the like. Furthermore, as shown in Table 3, these expression markers were still expressed after 25 passages (100 days).

### [Example 6] Examination of medium components suitable for culturing fish germ stem cells - 4

The conditions that enable maintenance and expansion culture of fish germ stem cells were examined. The killifish germ stem cells prepared in Experimental Example 1 were used. As the medium, DMEM/F12 (Ham) 1:1 medium was used as a basal medium. The presence or absence of the serum (10%, KSR (KnockOut^{™} Serum Replacement) Thermo fisher Scientific (GIBCO)), Chub mackerel plasma (1%, preparation method of plasma is as follows: Blood of Chub mackerel was collected by cardiac puncture using a 23-gauge needle and syringe treated with 0.25% disodium EDTA, the blood was promptly centrifuged at 6000 rpm for 10 min and the supernatant was collected. Plasma was UV irradiated for 5 min before use and filtered through a 0.22 µ filter. The plasma used in this Example was prepared by mixing plasma collected from Chub mackerel of various fish ages and sexes), vitronectin (human-derived, Thermo fisher Scientific (GIBCO)), and 8 specific components (substituted by E8 supplement, insulin, etc. are recombinant peptides and of human origin) was examined. Culture conditions were the same as in Example 1. The results are shown in Fig. 9.

As shown in Fig. 9, it was shown that germ stem cells can be cultured in a vitronectin-coated container and in a medium containing the E8 supplement, and serum and plasma are not always necessary. In other words, it was shown that fish germ stem cells can be cultured in a vitronectin-coated container and using a basal medium containing (1) insulin, (2) selenium, (3) transferrin, (4) L-ascorbic acid, (5) FGF2, (6) TGFβ, (7) NaHCO₃, and (8) L-glutamine.

### [Example 7] Examination of medium components suitable for culturing fish germ stem cells - 5

The germ stem cells of Chub mackerel prepared in Experimental Example 1 were cultured under various culture conditions 1 to 5 shown in Fig. 10, and the percentage of CD90.2-positive cells after culture was confirmed. The number of cells at the start of culture was 6.5×10⁵ cells or 25×10⁵ cells (the area of the culture dish used was 1.9 cm²). Respective conditions used such as culture temperature, CO₂ concentration, and the like were similar to those in Example 1. The media used in this Example were as follows:
DMEM-NAHCO3 (DMEM containing 1.743 g/L NaHCO₃ (see Table 1 for components other than DMEM))
DMEM-KHCO3 (DMEM containing 1.743 g/L KHCO₃ (see Table 1 for components other than DMEM))
L15-NAHCO3 (L15 containing 1.743 g/L NaHCO₃ (see Table 2 for components other than L15))

The results are shown in Fig. 10. As shown in Fig. 10, it was shown that NaHCO₃ can be replaced with KHCO₃ in the culture of fish germ stem cells.

### [Example 8] Examination of optimal cell density at the time of passage - 2

In the culture conditions of Example 7, whether the cell density at the time of passage influences the stemness and doubling time was examined. The germ stem cells of Chub mackerel prepared in Experimental Example 1 were used. The number of cells at the time of passage was 3.1×10⁵ cells, 6.25×10⁵ cells, 12.5×10⁵ cells, or 25×10⁵ cells (the area of the culture dish used was 1.9 cm²). The stemness and doubling time were determined in the same manner as in Example 4. The results are shown in Fig. 11.

As shown in Fig. 11, DMEM-NAHCO3 and DMEM-KHCO3 showed the same tendency in terms of stemness and doubling time, despite the cell density at the time of passage.

### [Example 9] Examination of medium components suitable for culturing fish germ stem cells - 6

The germ stem cells of Chub mackerel prepared in Experimental Example 1 were seeded in the two kinds of media (DMEM-NAHCO3 and DMEM-KHCO3) prepared in Example 7 at 3 cell densities, and the viability, confluency, and CFU (Colony forming Unit) 24 hr later were determined by the same method as in Example 3. In each experiment, N=6. The results are shown in Fig. 12.

As shown in Fig. 12, slight differences in the tendency sometimes occur due to differences in cell density at the time of seeding, but it was shown that both DMEM-NAHCO3 and DMEM-KHCO3 are suitable for culturing fish germ stem cells in terms of viability, confluency, and CFU. The media of "4" which gave the most favorable results from the results of Examples 7 to 9 (for detailed components, also refer to Table 1) are hereinafter referred to as "Media C" and "Media N". The "Media C" and "Media N" are specifically as follows:
Media C: DMEM/F12 (ham) 1:1+E8 supplement (excluding NaHCO₃)+NaHCO₃ (1.743 g/L)+L-ascorbic acid (64 mg/L) additionally added (total 128 mg/L)
Media N: DMEM/F12 (ham) 1:1+E8 supplement (excluding NaHCO₃)+KHCO₃ (1.743 g/L)+L-ascorbic acid (64 mg/L) additionally added (total 128 mg/L)

### [Example 10] Examination of medium components suitable for culturing fish germ stem cells - 7

The germ stem cells of killifish and Chub mackerel prepared in Experimental Example 1 were cultured under various culture conditions shown in Fig. 13, and the stemness and viability were determined 96 hr later. In Fig. 13, the "Medium" is DMEM, and serum, plasma, and vitronectin are all the same as those used in Example 6. The results are shown in Fig. 13.

As shown in Fig. 13, it was shown that the stemness and viability of the germ stem cells of killifish and Chub mackerel were fine when they were cultured using a vitronectin-coated container and Media C or Media N.

### [Example 11] Examination of optimal cell density at the time of passage - 3

The optimal cell density when the germ stem cells of Chub mackerel prepared in Experimental Example 1 are cultured using a vitronectin-coated container and Media C was examined. The cells were seeded at 3.1×10⁵ cells, 6.25×10⁵ cells, 12.5×10⁵ cells, or 25×10⁵ cells (the area of the culture dish used was 1.9cm²). The cell number, doubling time, confluency, stemness, and viability at the time points of 24 hr, 48 hr, 72 hr, and 96 hr after culture were determined. The results are shown in Fig. 14 and Fig. 15.

As shown in Fig. 14, when cells were seeded at a cell density of not less than 12.5×10⁵ cells/1.9 cm², the stemness and viability decreased. As shown in Fig. 14, when cells were seeded at a cell density of not less than 12.5×10⁵ cells/1.9 cm², some cell populations showed aggregation. From the above, it was shown that cell density at the time of passage is preferably relatively low.

### [Example 12] Examination of optimal cell density at the time of passage - 4

The optimal cell density of germ stem cells of Chub mackerel was examined under the same conditions as in Example 11 except that the medium used was changed to Media N. The cell number, doubling time, stemness, and viability at the time points of 24 hr, 48 hr, 72 hr, and 96 hr after culture were determined. The results are shown in Fig. 16.

As shown in Fig. 16, when cells were seeded at a cell density of not less than 12.5×10⁵ cells/1.9 cm², the stemness and viability decreased. Interestingly, the influence of cell density on the stemness and viability was greater by Medium N than Medium C.

### [Example 13] Improvement of DMEM-KHCO3 medium

As shown in Example 12, medium N (medium obtained by additionally adding ascorbic acid to DMEM-KHCO3) has lower tolerance with respect to cell density than Medium C (medium obtained by additionally adding ascorbic acid to DMEM-NAHCO3). When the aforementioned experiment was conducted, fish germ stem cells were cultured using DMEM-KHCO3 and there was a tendency for the cells to easily peel off from the surface of the culture container after about 2 weeks. Thus, the improvement of DMEM-KHCO3 was examined.

Medium N is composed of DMEM/F12 as a basal medium and further contains insulin, selenium, transferrin, L-ascorbic acid, FGF2, TGFβ, L-glutamine, KHCO₃, and HEPES (and gentamicin as antibiotic). The present inventors added various test substances to medium N and selected a substance suitable for the improvement of medium N. The standard of selection used were stemness, viability, mortality, confluency (2 days later), and colony number (4 days later). The germ stem cells of Chub mackerel were used. The test period was set to 15 days. In the test, N=6. The stemness, viability, mortality, confluency, and colony number were calculated with the results in Media C as the baseline.

As a result of selection, it was found that the addition of NaCl can favorably improve Medium N. The results with the addition of NaCl are shown in Fig. 17. In Fig. 17, "FSC10" is Media N supplemented with 60.9 µg/L sodium selenite (Na₂SeO₃) (21 µg/L converted to selenium concentration) and 121 mg/L NaCl. E8 is E8 medium (DMEM/F12 (Ham) (1:1) (ph 7.4) containing insulin 19.4 mg/L, selenium 14 µg/L, transferrin 10.7 mg/L, L-ascorbic acid 64 mg/L, FGF2 100 µg/L, TGFβ- 2 µg/L, L-glutamine 100 mg/L, NAHCO₃1.743 g/L, and HEPES 15 mM).

As shown in Fig. 17, FSC10 afforded good results as compared with Media N, in all standards of stemness, viability, mortality rate, confluency, and colony number.

### [Example 14] Examination of concentration range of components contained in FSC10 medium

Preferred concentration ranges of the components contained in FSC10 were examined. The germ stem cells of killifish and Chub mackerel were used. The test period was set to 7 days. In the test, N=10. The stemness, viability, confluency, and CFU were used as the standard. Concentration ranges were determined where each standard was at least 70% or greater as compared with FSC10 prepared in Example 13. The results are shown in Fig. 18.

### [Industrial Applicability]

According to the present invention, fish germ cells with stable quality can be proliferated efficiently at a comparatively low cost. Therefore, the present invention is extremely useful in the field of test research targeting fish and the field of production and breeding of fish, and the like.

This application is based on a patent application No. 2021-196963 filed in Japan (filing date: December 3, 2021), the contents of which are incorporated in full herein.

## Claims

1. A method for culturing a fish germ stem cell, comprising culturing the fish stem cell on a surface coated with vitronectin and in a medium comprising the following 8 components:
(1) insulin,
(2) selenium,
(3) transferrin,
(4) L-ascorbic acid,
(5) FGF2,
(6) TGFβ,
(7) NaHCO₃ or KHCO₃,
(8) L-glutamine.

2. The method according to claim 1, wherein the medium is a serum-free medium.

3. The method according to claim 1 or 2, wherein the method is performed under feeder-free conditions.

4. The method according to any one of claims 1 to 3, comprising a step of reseeding at 0.5×10⁵ to 6.5×10⁵ cells/cm² at the time of passage.

5. A kit for culturing a fish germ stem cell, comprising the following:
vitronectin, and
the following 8 components:
(1) insulin,
(2) selenium,
(3) transferrin,
(4) L-ascorbic acid,
(5) FGF2,
(6) TGFβ,
(7) NaHCO₃ or KHCO₃,
(8) L-glutamine.

6. The kit according to claim 5, further comprising a basal medium.

7. The kit according to claim 5 or 6, further comprising sodium chloride when (7) is KHCO₃.
